(19) Europäisches Patentamt
European Patent Office
Office européen des brevets

(11) Veröffentlichungsnummer: **0 027 906**
**B1**

(12) # EUROPÄISCHE PATENTSCHRIFT

(45) Veröffentlichungstag der Patentschrift: **25.05.83**

(21) Anmeldenummer: **80105872.8**

(22) Anmeldetag: **27.09.80**

(51) Int. Cl.³: **C 07 C 143/72,** C 07 C 143/74, C 07 C 143/78, C 07 C 87/30, C 07 C 91/04

(54) **Verfahren zur Herstellung von Sulfonamiden.**

(30) Priorität: **13.10.79 DE 2941593**

(43) Veröffentlichungstag der Anmeldung: **06.05.81 Patentblatt 81/18**

(45) Bekanntmachung des Hinweises auf die Patenterteilung: **25.05.83 Patentblatt 83/21**

(84) Benannte Vertragsstaaten: **BE CH DE FR GB IT LI NL**

(56) Entgegenhaltungen:
**US-A-2 334 186**
**Patents Abstracts of Japan, Band 2, Nr. 38, 14. März 1978, Seite 4598C77**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Staffe, Adolf, Dr., Erfurter Strasse 5, D-5090 Leverkusen 1 (DE)**

**EP 0 027 906 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von Sulfonamiden

Die vorliegende Erfindung betrifft ein Verfahren zur Herstellung von Sulfonamiden durch Umsetzung von Sulfonsäurehalogeniden mit Ammoniak oder primären oder sekundären Aminen.

Es ist bekannt, Sulfonsäureamide durch Umsetzung von aliphatischen oder stark negativ substituierten aromatischen Aminen oder Ammoniak mit Sulfochloriden herzustellen (vgl. Houben – Weyl, Methoden der organischen Chemie, 4. Auflage, Band IX, Seiten 609 – 614).

Dabei kann die Umsetzung in Wasser vorgenommen werden, wenn das Amin zumindest in geringem Maße in Wasser löslich ist. Wenn das Amin in Wasser unlöslich ist, wird die Umsetzung in einem organischen Lösungsmittel, das nicht unbedingt mit Wasser mischbar sein muß, durchgeführt.

Die bekannten Verfahren zur Herstellung von Sulfonamiden weisen jedoch nicht unerhebliche Nachteile auf. Sie erfordern z. B. hohe Umsetzungsraten bei Anwesenheit organischer Lösungsmittel sehr lange Reaktionszeiten, was eine schlechte Raum/Zeit-Ausbeute ergibt oder die Ausbeuten an Sulfonamiden sind nicht optimal, was bei teuren Ausgangsmaterialien sehr kostspielig und damit für ein technisches Verfahren unwirtschaftlich ist.

Weiterhin ist es aus der JP-Anmeldung 51/54 673 bekannt, $N^2$-Alkoxynaphthalin-sulfonylarginin durch Umsetzung von Alkoxynaphthalin-sulfonyl-halogenid mit Arginin in Wasser oder in wäßrig-organischer Mischphase herzustellen. Hierbei wird in Gegenwart von organischen oder anorganischen Basen in mindestens äquivalenter Menge, bezogen auf das Arginin gearbeitet, um den abgespaltenen Halogenwasserstoff aufzufangen.

Es wurde nun ein Verfahren zur Herstellung von Sulfonamiden durch Umsetzung von Sulfonsäurehalogeniden mit Ammoniak oder primären oder sekundären Aminen gefunden, das dadurch gekennzeichnet ist, daß man die Reaktion in einem Zweiphasensystem, bestehend aus einer wäßrigen Phase und einer organischen Phase, in Gegenwart von 0,1 bis 12 Gew.-% eines tertiären Amins und/oder eines quarternären Ammoniumsalzes, bezogen auf das eingesetzte Sulfonsäurehalogenid, durchführt.

Als quarternäre Ammoniumsalze können solche der allgemeinen Formel (I)

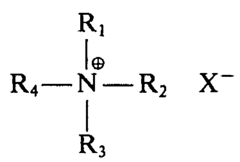

(I)

in der

$R_1$, $R_2$, $R_3$ und $R_4$ unabhängig voneinander einen gegebenenfalls substituierten Alkyl-, Aralkyl- oder Arylrest bedeuten oder wobei jeweils zwei der Reste $R_1$, $R_2$, $R_3$ und $R_4$ gemeinsam mit dem Stickstoff einen Heterocyclus bilden und
$X^-$ für ein Halogen- oder Hydroxyl-Ion steht,
in das das erfindungsgemäße Verfahren eingesetzt werden.

Als Alkylreste, die gegebenenfalls durch Halogen, wie Chlor, Brom, eine Hydroxy- oder niedere Alkoxygruppe mit bis zu 4 Kohlenstoffatomen, wie Hydroxybutyl, substituiert sein können, kommen beispielsweise solche mit bis zu 16, bevorzugt bis zu 12 Kohlenstoffatomen in Betracht.

Als gegebenenfalls substituierte Alkylreste seien genannt: Methyl, Ethyl, Propyl, Butyl, Pentyl, Hexyl, Heptyl, Octyl, Nonyl, Decyl, Undecyl, Dodecyl, Hydroxyethyl, Hydroxypropyl, Hydroxybutyl, Chlormethyl, Chlorethyl, Chlorbutyl, Chlorpropyl, bevorzugt Methyl, Ethyl, Propyl, Butyl, Dodecyl, Hydroxyethyl, Hydroxypropyl, Hydroxybutyl.

Als Aralkylreste, die gegebenenfalls durch Halogen, wie Chlor, Brom, oder Alkylreste mit bis zu 4 Kohlenstoffatomen substituiert sein können, kommen beispielsweise solche mit bis zu 10 Kohlenstoffatomen, bevorzugt bis zu 8 Kohlenstoffatomen, in Betracht.

Es seien genannt: Phenylmethyl-, Phenylethyl-, Phenylpropyl-, Phenylbutyl-, (Chlorphenyl)-methyl-, (Methylphenyl)-methyl-, (Methylphenyl)-ethylreste, bevorzugt Phenylmethylreste.

Als Arylreste, die gegebenenfalls durch Halogen, wie Chlor, Brom, oder Alkylreste mit bis zu 4 Kohlenstoffatomen, wie Methyl, Ethyl, Propyl, Butyl, i-Propyl, I-Butyl, Dimethyl, Diethyl, Chlor-methyl, substituiert sein können, kommen beispielsweise solche mit bis zu 14 Kohlenstoffatomen, bevorzugt bis zu 12 Kohlenstoffatomen, in Betracht.

Als Arylreste seien genannt: Phenyl, Chlorphenyl, Methylphenyl, Ethylphenyl, Propylphenyl, Butylphenyl, Dimethylphenyl, Chlormethylphenyl, Naphthyl, Dimethylnaphthyl, bevorzugt Phenyl.

Als Heterocyclen, die jeweils 2 benachbarte Reste $R_1$, $R_2$, $R_3$, $R_4$ zusammen mit dem Stickstoffatom bilden können, seien vor allem der Piperidin- oder Morpholinring erwähnt.

Als Halogenionen seien genannt: das Fluor-, Chlor-, Brom-, Jodion, bevorzugt das Chlorion.

Als Vertreter der quartären Ammoniumsalze, die in das erfindungsgemäße Verfahren eingesetzt werden können, werden beispielsweise genannt: Trimethylphenylammoniumchlorid, Trimethylbenzylammoniumchlorid, Trimethylbenzylammoniumhydroxid, Triethylammoniumchlorid, Tetraethylammoniumchlorid, Dimethylphenylbenzylammoniumchlorid, Tetrabutylammoniumchlorid, Benzyldodecyldimethylammoniumchlorid, Triethylbenzylammoniumchlorid, Benzyldodecyldihydroxypropylammoniumchlorid, bevorzugt Benzyldodecyldimethylammoniumchlorid.

Als tertiäre Amine können solche der allgemeinen Formel (II)

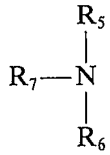

(II)

in der

$R_5$, $R_6$, $R_7$ unabhängig voneinander für einen gegebenenfalls substituierten Alkyl-, Cycloalkyl-, Aralkyl- oder Arylrest stehen oder jeweils zwei der Reste $R_5$, $R_6$, $R_7$ zusammen mit dem Stickstoffatom einen Heterocyclus bilden,

in das erfindungsgemäße Verfahren eingesetzt werden.

Als Alkylreste, die gegebenenfalls durch eine Hydroxy- oder Alkoxygruppe mit bis zu 4 Kohlenstoffatomen, wie Hydroxyethyl, Hydroxypropyl oder Hydroxybutyl substituiert sein können, kommen beispielsweise solche mit bis zu 16 Kohlenstoffatomen, bevorzugt bis zu 12 Kohlenstoffatomen in Betracht.

Es seien folgende Alkylreste genannt: Methyl, Ethyl, Propyl, Butyl, Pentyl, Isopropyl, 2-Hydroxyethyl, 2-Hydroxypropyl, Hexyl, Octyl, Decyl, Dodecyl, 4-Hydroxyethylpropyl, i-Butyl, i-Pentyl, bevorzugt Methyl, Ethyl, 2-Hydroxyethyl, Propyl, 2-Hydroxypropyl, Hexyl, Octyl, Dodecyl, I-Butyl, i-Pentyl.

Als Aralkylreste, die gegebenenfalls durch Halogen, wie Chlor, Brom, oder Alkylreste mit bis zu 4 Kohlenstoffatomen substituiert sein können, kommen beispielsweise solche mit bis zu 10 Kohlenstoffatomen, bevorzugt bis zu 8 Kohlenstoffatomen in Betracht.

Es werden z. B. genannt: Phenylmethyl-, (Chlor-phenyl)-methyl-, (Methylphenyl)-methylreste, bevorzugt der Phenylmethylrest.

Als Arylreste, die gegebenenfalls durch Halogen, wie Chlor, Brom, oder Alkylreste mit bis zu 4 Kohlenstoffatomen substituiert sein können, kommen beispielsweise solche mit bis zu 14 Kohlenstoffatomen, bevorzugt 10 Kohlenstoffatomen in Frage, wie der Phenyl-, Chlorphenyl-, Methylphenyl-, Dimethylphenyl-, Naphthylrest, bevorzugt der Phenylrest.

Als Heterocyclen, die jeweils zwei benachbarte Reste $R_5$, $R_6$, $R_7$ zusammen mit dem Stickstoffatom bilden können, seien vor allem der Piperidin- oder der Morpholinring erwähnt.

Als Vertreter der tertiären Amine, die in das erfindungsgemäße Verfahren eingesetzt werden können, seien genannt: Trimethylamin, Triethylamin, Tris-(2-hydroxyethyl)-amin, Tri-iso-propylamin, Tris-(2-hydroxypropyl)-amin, Tri-n-propylamin, Tri-n-butylamin, Tri-iso-butylamin, Tri-n-pentylamin, Tri-iso-pentylamin, Dimethylanilin, Diethylanilin, Dimethylbenzylamin, bevorzugt Tris(2-hydroxypropyl)-amin.

Die Einsatzmengen für die tertiären Amine und/oder quartären Ammoniumsalze können in weiten Grenzen schwanken. Sie betragen im allgemeinen etwa 0,1 bis etwa 12 Gew.-%, vorzugsweise 0,5 bis 5,5 Gew.-%, bezogen auf das Gewicht der eingesetzten Sulfonsäurehalogenide.

Die tertiären Amine und/oder quartären Ammoniumsalze können gelöst oder suspendiert in Wasser dem Reaktionssystem zugeführt werden.

Als Sulfonsäurehalogenide, die die eine Reaktionskomponente für das erfindungsgemäße Verfahren darstellen, können solche der allgemeinen Formel (III)

$$R_8-SO_2-Hal \qquad (III)$$

in der

Hal für Fluor, Chlor, Brom, bevorzugt Chlor, steht und

$R_8$ für einen gegebenenfalls substituierten Alkyl-, Alkenyl-, Cycloalkyl-, Aralkyl- oder Arylrest steht,

in das erfindungsgemäße Verfahren eingesetzt werden.

Als Alkylreste, die gegebenenfalls durch Halogen, wie Fluor, Chlor, substituiert sein können, kommen beispielsweise solche mit bis zu 24 Kohlenstoffatomen, bevorzugt bis zu 18 Kohlenstoffatomen in Betracht.

Genannt seien: Methyl-, Chlormethyl-, Dichlormethyl-, Trichlormethyl-, Trifluormethyl-, Ethyl-, Chlorethyl-, Propyl-, Butyl-, Chlorbutyl-, Hexyl-, Heptyl-, Octyl-, Undecyl-, Dodecyl-, Pentadecyl-Hexadecylreste, Gemische von $C_{10}-C_{18}$ n-Alkylresten, Perfluorbutyl-, Perfluoroctylreste, bevorzugt Methyl-, Chlormethyl-, Butyl-, Perfluorbutyl-, Octyl-, Perfluoroctyl-, Undecyl-, Dodecyl-, Pentadecyl-Hexadecylreste und Gemische von $C_{10}-C_{18}$ n-Alkylresten.

Als Alkenylreste, die gegebenenfalls durch Halogen, wie Chlor, Brom, substituiert sein können, kommen beispielsweise solche mit bis zu 10 Kohlenstoffatomen, bevorzugt bis zu 6 Kohlenstoffatomen in Betracht.

Es seien als Alkenylreste genannt: Vinyl, 2-Propenyl, 2-Butenyl, 2-Pentenyl, 2-Hexenyl, bevorzugt Vinyl.

Als Cycloalkylreste, die gegebenenfalls durch Methylgruppen substituiert sein können, kommen beispielsweise solche mit bis zu 10 Kohlenstoffatomen, bevorzugt bis zu 8 Kohlenstoffatomen in Betracht.

Zum Beispiel werden genannt: Cyclopropyl-, Cyclobutyl-, Cyclopentyl-, Cyclohexyl-, Cycloheptyl-, Cyclooctyl-, Methylcyclopentyl-, Methylcyclohexyl-, Dimethylcyclohexylreste, bevorzugt Cyclohexyl-, Methylcyclohexyl-, Cyclooctylreste.

Als Aralkylreste, die gegebenenfalls durch Halogen, wie Chlor, Brom, substituiert sein können, kommen beispielsweise solche mit bis zu 18 Kohlenstoffatomen, bevorzugt bis zu 10 Kohlenstoffatomen in Betracht.

Zum Beispiel seien genannt: Phenylmethyl-, (Chlorphenyl)-methyl-, (Trifluormethylphenyl)-methyl-, (Trimethylphenyl)-methylreste, bevorzugt der Phenylmethylrest.

Als Arylreste, die gegebenenfalls durch Halogen, wie Chlor, Brom, oder Sulfochloridgruppen oder Carboxylgruppen substituiert sein können, kommen beispielsweise solche mit bis zu 14 Kohlenstoffatomen, bevorzugt bis zu 12 Kohlenstoffatomen in Betracht.

Zum Beispiel werden genannt: der Phenyl-, Chlorphenyl-, Dimethylnaphthyl-, Naphthyl-, Methylphenyl-, Dimethylphenyl-, Naphthylsulfochlorid-, Phthalsäurerest, bevorzugt der Phenylrest.

Zum Beispiel seien folgende Sulfonsäurehalogenide, die in das erfindungsgemäße Verfahren eingesetzt werden können, genannt: Methansulfonsäurechlorid, Chlormethansulfonsäurechlorid, Dichlormethansulfonsäurechlorid, Trichlormethansulfonsäurechlorid, Ethansulfonsäurechlorid, Vinylsulfonsäurechlorid, Chlorethansulfonsäurechlorid, Propansulfonsäurechlorid, Butansulfonsäurechlorid, Chlorbutansulfonsäurechlorid, Pentansulfonsäurechlorid, Hexansulfonsäurechlorid, Heptansulfonsäurechlorid, Octansulfonsäurechlorid, Undecansulfonsäurechlorid, Dodecansulfonsäurechlorid, Pentadecansulfonsäurechlorid, Stearinsulfonsäurechlorid, Gemische von $C_{10}-C_{18}$-n-Alkansulfonsäurechloride, Dimethylvinylsulfonsäurechlorid, Methallylsulfonsäurechlorid, Phenylmethansulfonsäurechlorid, 1,2-Phenylen-bis-methan-sulfonsäurechlorid, Trichlorvinylsulfonsäurechlorid, Benzolsulfonsäurechlorid, Chlorbenzolsulfonsäurechlorid, Dichlorbenzolsulfonsäurechlorid, Brombenzolsulfonsäurechlorid, Fluorbenzolsulfonsäurechlorid, Jodbenzolsulfonsäurechlorid, Nitrobenzolsulfonsäurechlorid, Dinitrobenzolsulfonsäurechlorid, Toluolsulfonsäurechlorid, Trifluormethylbenzolsulfonsäurechlorid, Naphthalin-1-sulfonsäurechlorid, Naphthalin-2-sulfonsäurechlorid, Methoxysulfonsäurechlorid, Acetaminobenzolsulfonsäurechlorid, Chlornitrobenzolsulfonsäurechlorid, Nitrotoluolsulfonsäurechlorid, Dinitrotoluolsulfonsäurechlorid, Tetralinsulfonsäurechlorid, Nitro-naphthalinsulfonsäurechlorid, Biphenylsulfonsäurechlorid, Pyrensulfonsäurechlorid, Benzoldisulfonsäurechlorid, Nitrobenzoldisulfonsäurechlorid, Naphthalindisulfonsäurechlorid, Dinitro-naphthalindisulfonsäurechlorid, Biphenyldisulfonsäurechlorid, Naphthalintrisulfonsäurechlorid, Naphthalintetrasulfonsäurechlorid, Diphenylsulfon-disulfonsäurechlorid, Diphenylsulfon-disulfonsäurechlorid, Diphenylethyl-disulfonsäurechlorid, Nitroanisolsulfonsäurechlorid, Anthrachinonsulfonsäurechlorid, Benzoesäuresulfonsäurechlorid, Chlorbenzoesäuresulfonsäurechlorid, Benzoesäuredisulfonsäurechlorid, Phthalsäuresulfonsäurechlorid, Terephthalsäuresulfonsäurechlorid, Trifluormethansulfonsäurefluorid, Perfluorbutansulfonsäurefluorid, Perfluoroctansulfonsäurefluorid, bevorzugt Chlormethansulfonsäurechlorid, Undecansulfonsäurechlorid, Pentadecansulfonsäurechlorid und Gemische von $C_{10}-C_{18}$-n-Alkansulfonsäurechloriden.

Ammoniak sowie die primären und sekundären Amine, die die andere Reaktionskomponente für das erfindungsgemäße Verfahren darstellen, können durch die allgemeine Formel (IV)

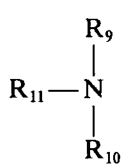

(IV)

in der

$R_9$ für Wasserstoff und

$R_{10}$, $R_{11}$ unabhängig voneinander für Wasserstoff oder einen gegebenenfalls substituierten Alkyl-, Alkenyl-, Cycloalkyl-, Aralkyl-, Aryl-, Diarylether- oder Arylalkyletherrest stehen oder worin die Reste $R_{10}$ und $R_{11}$ zusammen mit dem Stickstoff und gegebenenfalls weiteren Heteroatomen, wie Sauerstoff, Schwefel Stickstoff, einen Heterocyclus bilden,

dargestellt werden.

Als Alkylreste, die gegebenenfalls durch Halogen, wie Chlor, Brom, Hydroxy-, Amino- oder niedere

Alkoxygruppen mit bis zu 4 Kohlenstoffatomen, wie 2-Hydroxybutyl, substituiert sein konnen, kommen beispielsweise solche mit bis zu 18 Kohlenstoffatomen, bevorzugt bis 16 Kohlenstoffatomen in Betracht.

Zum Beispiel werden genannt: der Methyl-, Ethyl-, Propyl-, i-Propyl-, Butyl-, Pentyl-, Hexyl-, Octyl-, Dodecyl-, Octadecyl-, 1-Aminobutyl-, 1-Aminohexyl-, 1-Chlorethyl-, 1-Chlorpropyl-, 1-Fluorbutyl-, 1-Oxyethyl-, 2-Oxypropyl-, N,N-Dimethyl-1-aminopropylrest, bevorzugt der Methyl-, Ethyl-, Propyl-, Hexyl-, Octyl-, Octadecyl-, 1-Aminobutyl-, 1-Aminohexylrest.

Als Alkenylreste, die gegebenenfalls durch Halogen, wie Chlor, Brom, substituiert sein können, kommen beispielsweise solche mit bis zu 18 Kohlenstoffatomen, bevorzugt bis zu 6 Kohlenstoffatomen in Betracht.

Zum Beispiel werden genannt: der 2-Propenyl-, 2-Butenyl-, 2-Pentenyl-, 2-Hexenyl-, 2-Cyclohexenylrest, bevorzugt der 2-Propenyl- und 2-Hexenylrest.

Als Cycloalkylreste, die gegebenenfalls durch Methylgruppen substituiert sein können, kommen beispielsweise solche mit bis zu 10 Kohlenstoffatomen, bevorzugt bis zu 8 Kohlenstoffatomen in Betracht.

Genannt seien: Cyclopropyl-, Cyclobutyl-, Cyclopentyl-, Cyclohexyl-, Cycloheptyl-, Cyclooctyl-, Methylcyclopentyl-, Methylcyclohexyl-, Dimethylcyclohexylreste, bevorzugt der Cyclohexyl- und Methylcyclohexylrest.

Als Aralkylreste, die gegebenenfalls durch Halogen, wie Chlor, Brom, Aminogruppen, Alkylreste mit bis zu 12 Kohlenstoffatomen, wie Dodecyl, oder Alkoxygruppen mit bis zu 3 Kohlenstoffatomen, wie 2-Hydroxypropyl, substituiert sein können, kommen beispielsweise solche mit bis zu 18 Kohlenstoffatomen, bevorzugt 11 Kohlenstoffatomen in Betracht.

Folgende Aralkylreste seien genannt: der Phenylmethyl-, (Chlorphenyl)-methyl-, Naphthylmethylrest, bevorzugt der Phenylmethylrest.

Als Arylreste, die gegebenenfalls durch Halogen, wie Chlor, Brom, Trifluormethylreste, Alkylreste mit bis zu 12 Kohlenstoffatomen, wie Dodecyl, Cycloalkylreste mit bis zu 6 Kohlenstoffatomen, wie Cyclohexyl, Alkoxygruppen mit bis zu 3 Kohlenstoffatomen, wie Propoxy, oder Aminogruppen substrahiert sein können, kommen beispielsweise solche mit bis zu 20 Kohlenstoffatomen, bevorzugt bis zu 18 Kohlenstoffatomen in Betracht.

Zum Beispiel werden folgende Arylreste genannt: Phenyl, 2-Chlorphenyl, 3-Chlorphenyl, 4-Chlorphenyl, 3,4-Dichlorphenyl, 3-Chlor-2-methylphenyl, 2-Methylphenyl, 3-Methylphenyl, 2-Ethylphenyl, 4-Ethylphenyl, 2,3-Dimethylphenyl, Dodecylphenyl, 1-Naphthyl, 2-Amino-4-chlorphenyl, 4-Aminophenyl, 4-Amino-1-naphthyl, 3-Trifluormethylphenyl, 5-Amino-1-naphthyl, 3-Amino-2-naphthyl, 4-Cyclohexyl-phenyl, 4-Cyclohexyl-2-methylphenyl, bevorzugt Phenyl, 3-Chlorphenyl, 2-Chlorphenyl, 1-Naphthyl.

Als Diaryletherreste kommen beispielsweise solche mit bis zu 18 Kohlenstoffatomen, bevorzugt bis zu 16 Kohlenstoffatomen in Betracht.

Genannt seien: der 2-Phenoxyphenyl-, 2-Phenoxy-2′-methylphenyl-, 2-Phenoxy-naphthylrest, bevorzugt der 2-Phenoxyphenylrest.

Als Arylalkyletherreste kommen beispielsweise solche mit bis zu 18 Kohlenstoffatomen, bevorzugt bis zu 11 Kohlenstoffatomen in Betracht, z. B. der 4-Methoxyphenyl-, 2-Ethoxyphenyl-, 4-Methoxyphenyl-, 4-Ethoxyphenyl-, 2-Methoxynaphthyl-, 4-Methoxynaphthylrest bevorzugt der 2-Methoxyphenyl- und 2-Methoxynaphthylrest.

Als Heterocyclen, die $R_{10}$ und $R_{11}$ zusammen mit dem Stickstoff und gegebenenfalls weiteren Heteroatomen, wie Sauerstoff, Schwefel, Stickstoff, bilden können, seien der Pyrrolidin-, Piperidin- oder Morpholinring erwähnt.

Zum Beispiel werden folgende Amine, die in das erfindungsgemäße Verfahren eingesetzt werden können, genannt: Ammoniak, Methylamin, Dimethylamin, Ethylamin, Diethylamin, n-Propylamin, Di-n-propylamin, i-Propylamin, Butyl-, Pentyl-, Hexyl-, Octyl-, Dodecyl-, Octadecylamin, Dibutylamin, Dipentylamin, Dihexylamin, Dioctylamin, Didodecylamin, Dioctadecylamin, Allylamin, Cyclohexylamin, Benzylamin, N-Methylbenzylamin, N-Ethylbenzylamin, Anilin, N-Methylanilin, N-Ethylanilin, 2-Chloranilin, 3-Chloranilin, 4-Chloranilin, 2,3-Dichloranilin, 3,4-Dichloranilin, 4-Chlor-2-methylanilin, 3-Chlor-2-methylanilin, 2-Methylanilin, 4-Methylanilin, 3-Methylanilin, 3-Methylanilin, 2-Ethylanilin, 4-Ethylanilin, 2,3-Dimethylanilin, 4-Cyclohexylanilin, 1-Naphthylamin, N-Ethyl-1-naphthylamin, 2-Naphthylamin, N-Ethyl-2-naphthylamin, 2-Aminodiphenylether, 2-Amino-2′-methyldiphenylether, 2-Aminophenyl-1-naphthylether, 2-Aminophenyl-2-naphthylether, 2-Amino-1-ethoxybenzol, 2-Amino-1-methoxybenzol, 1,2-Diaminobenzol, 4-Chlor-1,2-diaminobenzol, 3-Amino-ethylanilin, 1,4-Diaminobenzol, N-Phenyl-1,4-benzoldiamin, 2-Chlor-1,4-diaminobenzol, 1,3-Diamino-4-methylbenzol, 1,4-Diaminonaphthalin, 1,5-Diaminonaphthalin, 2,7-Diaminonaphthalin, 4,4′-Diaminodiphenylether, Morpholin, Tetramethylendiamin, bevorzugt Ammoniak, Anilin, 2-Chloranilin, 3-Chloranilin und 2-Aminodiphenylether.

Die organische Phase wird bei dem erfindungsgemäßen Verfahren von dem Sulfonsäurehalogenid und dem Sulfonamid bzw. der Lösung des Sulfonsäurehalogenids und des Sulfonamids in einem unter den Reaktionsbedingungen inerten und mit Wasser nur wenig mischbaren organischen Lösungsmittel gebildet.

Als inerte organische Lösungsmittel, die mit Wasser nur wenig mischbar sind, kann man z. B. n-Alkane mit 5 bis 20 Kohlenstoffatomen, bevorzugt 6 bis 18 Kohlenstoffatomen, in das erfindungsgemäße Verfahren einsetzen.

Beispielsweise werden folgende n-Alkane genannt: n-Pentan, n-Hexan, n-Heptan, n-Octan, n-Nonan, n-Decan, n-Undecan, n-Dodecan, n-Tetradecan, n-Pentadecan, n-Hexadecan, n-Heptadecan, n-Octadecan, bevorzugt n-Decan, n-Undecan, n-Dodecan, n-Tetradecan, n-Pentadecan, n-Hexadecan.

Es ist auch möglich Cycloalkane mit bis zu 7 Kohlenstoffatomen, wie Cyclohexan oder Cycloheptan; aromatische Kohlenwasserstoffe mit bis zu 10 Kohlenstoffatomen, bevorzugt bis zu 8 Kohlenstoffatomen, wie Benzol, Toluol oder Xylol und chlorierte aliphatische oder aromatische Kohlenwasserstoffe mit bis zu 10 Kohlenstoffatomen, bevorzugt bis zu 8 Kohlenstoffatomen, wie Chlorbenzol, Dichlorbenzol, Trichlorbenzol, Dichlortoluol, Trichlortoluol, Chlortoluol, Methylenchlorid, Chloroform, Tetrachlorkohlenstoff, 1,2-Dichlorethan, 1,2-Dichlorpropan, Tetrachlorethan oder Trichlorethan in das erfindungsgemäße Verfahren einzusetzen.

Bevorzugt werden Cyclohexan, Toluol, Dichlorbenzol, 1,2-Dichlorethan und 1,2-Dichlorpropan eingesetzt.

Außerdem können Benzin (Siedepunkt 60 – 95° C) und Petrolether (Siedepunkt 40 – 80° C) in das erfindungsgemäße Verfahren eingesetzt werden.

Die inerten organischen Lösungsmittel können sowohl einzeln als auch im Gemisch untereinander in das erfindungsgemäße Verfahren eingesetzt werden.

Die wäßrige Phase wird bei dem erfindungsgemäßen Verfahren von der wäßrigen Lösung oder Suspension der tertiären Amine und/oder quartären Ammoniumsalze gebildet. Es ist wichtig, daß zumindest ein Teil des als Umsetzungspartner eingesetzten Ammoniaks oder primären oder sekundären Amins in der wäßrigen Phase gelöst ist. Unter Umständen kann also die wäßrige Phase auch noch ungelöstes primäres oder sekundäres Amin enthalten. Die Einsatzmengen an Ammoniak oder primären oder sekundären Aminen und Sulfonsäurehalogeniden können in weiten Grenzen schwanken. Zum Beispiel können auf 1 Mol Ammoniak oder primären oder sekundären Aminen etwa 0,1 bis etwa 5 Mol, vorzugsweise 0,25 bis 2,5 Mol, Sulfonsäurehalogenid eingesetzt werden.

Bei dem erfindungsgemäßen Verfahren kann der für die Bildungsreaktion der Sulfonamide optimale pH-Bereich in den Grenzen von etwa 0,5 bis etwa 12,5, vorzugsweise im Bereich von 1 bis 10,5, schwanken und muß für jedes eingesetzte Amin bestimmt und dann während der gesamten Umsetzungszeit konstant gehalten werden. Wird die Umsetzung bei einem pH-Wert durchgeführt, der nicht dem optimalen pH-Wert für das entsprechende Amin entspricht, so erhält man niedrigere Sulfonamid-Ausbeuten.

Das erfindungsgemäße Verfahren kann bei Temperaturen im Bereich von etwa –15 bis etwa +180° C, vorzugsweise 0 bis 80° C, besonders bevorzugt bei 5 bis 60° C durchgeführt werden.

Das erfindungsgemäße Verfahren kann bei Unterdruck (–0,1 bis 0,9 bar, bevorzugt –0,2 bis –0,5 bar), Normaldruck und Überdruck (0,1 bis 6 bar, bevorzugt 0,1 bis 0,5 bar) durchgeführt werden. Vorzugsweise arbeitet man bei Normal- oder Überdruck.

Bei dem erfindungsgemäßen Verfahren hängt die Reaktionsdauer von der Reaktivität der eingesetzten primären oder sekundären Amine und Sulfonsäurehalogenide ab und liegt erfahrungsgemäß bei etwa 10 Minuten bis etwa 20 Stunden. Daraus ergibt sich, daß das Verfahren diskontinuierlich als auch kontinuierlich durchgeführt werden kann. Vorteilhafterweise werden beim erfindungsgemäßen Verfahren bei kontinuierlicher Fahrweise alle wieder einsetzbaren Ausgangsprodukte im Kreise geführt.

Das erfindungsgemäße Verfahren kann folgendermaßen durchgeführt werden: Zu einer wäßrigen Lösung aus einem tertiären Amin und/oder quartären Ammoniumsalz wird das in verdünnter Salzsäure teilweise gelöste primäre oder sekundäre Amin zugesetzt. Vorher hat man den für das primäre oder sekundäre Amin optimalen pH-Wert in einigen Vorversuchen ermittelt. Der ermittelte pH-Wert wird nun in der wäßrigen Phase durch Zugabe von Sodalösung eingestellt und auch während der nachfolgenden Zugabe einer Lösung von Sulfonsäurehalogenid in einem inerten mit Wasser kaum mischbaren organischen Lösungsmittel gegebenenfalls durch weitere Zugabe von Sodalösung gehalten. Man läßt die beiden Reaktionspartner so lange reagieren, bis kein Sodaverbrauch mehr bei konstantem pH-Wert auftritt. Anschließend wird die Reaktionsmischung erwärmt, um den Überschuß an Sulfonsäurehalogenid zu verseifen. Um das gesamte Reaktionsprodukt in Lösung zu bringen, setzt man dem Reaktionsgemisch noch weiteres Lösungsmittel zu. Zur Entfernung von unumgesetztem Amin extrahiert man zuerst die organische Phase mit verdünnter Salzsäure, dann mit Wasser, bis der pH-Wert der wäßrigen Phase etwa 6 erreicht hat. Zur Isolierung des Sulfonamids entfernt man das Lösungsmittel der organischen Phase durch Destillation im Vakuum.

Eine andere Variante des erfindungsgemäßen Verfahrens kann folgendermaßen durchgeführt werden: In einem Reaktionsgefäß wird eine Lösung aus einem tertiären Amin und/oder quartären Ammoniumsalz vorgelegt. Man evakuiert das Reaktionsgefäß und stellt anschließend durch Einleiten von Ammoniak einen leichten Überdruck von etwa 0,1 bis etwa 0,3 bar ein. Nun wird mit einer Dosierpumpe eine Lösung eines Sulfonsäurehalogenids in einem inerten, mit Wasser kaum mischbaren organischen Lösungsmittel zudosiert. Gleichzeitig hält man durch weitere Zugabe von

Ammoniak sowohl den Druck als auch den pH-Wert in einem engen alkalischen Bereich. Mit diesen pH- und Druckwerten wird die Dosiergeschwindigkeit und damit die Reaktionsgeschwindigkeit von Sulfonsäurehalogeniden gekoppelt. Zur Aufarbeitung wird die Reaktionsmischung unter Vakuum von überschüssigem Ammoniak befreit und zur Entfernung des gebildeten Ammoniumchlorids und kleiner Mengen Sulfonsäuren mehrmals mit verdünnter Kochsalzlösung und Wasser gewaschen. Das gebildete Sulfonamid liegt als Lösung vor und kann entweder direkt weiter verarbeitet oder nach Entfernen des Lösungsmittels durch Destillation im Vakuum isoliert werden.

Die Vorteile des erfindungsgemäßen Verfahrens gegenüber den literaturbekannten Verfahren liegen in den wesentlich höheren Ausbeuten und/oder den kürzeren Reaktionszeiten. Dadurch gestaltet sich das erfindungsgemäße Verfahren besonders wirtschaftlich.

Die nach dem erfindungsgemäßen Verfahren hergestellten Sulfonamide können zur Herstellung von Entschäumern oder als Zwischenprodukte für Textilausrüstungsmittel, Farbstoffe, Pharmazeutika und Pflanzenschutzmittel (vgl. z. B. DE-PS 744 679, DE-PS 890 883) verwendet werden.

Die nachfolgenden Beispiele sollen das erfindungsgemäße Verfahren verdeutlichen, ohne es jedoch auf diese Beispiele einzuschränken.

## Beispiel 1

In einem 2-l-Vierhalskolben wurden 200 g Wasser, 25 g Salzsäure (36%ig) und 56,6 g 4-Chlor-2-methyl-anilin vorgelegt und 25 Minuten bei 40°C verrührt. Das Gemisch wurde auf 5°C abgekühlt und durch Zugabe von 17%iger Sodalösung auf einen pH-Wert von 2 eingestellt. Man setzte dem Gemisch 2,3 g einer 50%igen wäßrigen Lösung von Benzyldodecyldimethylammoniumchlorid zu und rührte ca. 15 Minuten. Bei einer Temperatur von 5°C wurde innerhalb von 2 Stunden eine Lösung von 49,2 g Chlormethansulfochlorid in 21,1 g Dichlorpropan zugetropft. Der pH-Wert wurde durch gleichzeitige Zudosierung von 17%iger Sodalösung bei einem Wert von 2 gehalten. Anschließend wurde bei 20°C noch 15 Stunden nachgerührt, wobei der pH-Wert durch weitere Zugabe von Sodalösung konstant gehalten wurde. Anschließend wurde das Reaktionsgemisch auf 70°C erwärmt und mit 100 ml Dichlorpropan versetzt. Die wäßrige Phase wurde abgetrennt und verworfen, die organische Phase wurde 5mal mit je 50 ml 15%iger Salzsäure und danach 2mal mit je 50 ml Wasser extrahiert. Dann entfernte man aus der organischen Phase durch Vakuumdestillation (ca. 50 mbar) bei 60°C das Lösungsmittel. Der Destillationsrückstand bestand aus 4-Chlor-2-methyl-N-(chlormethansulfo)-anilin (55,5 g) Reinheit: 98%; Ausbeute: 86,2% der Theorie.

## Vergleichsbeispiel

Es wurde analog Beispiel 1 gearbeitet, nur wurde dem Reaktionsgemisch kein Benzyldodecyldimethylammoniumchlorid zugesetzt. Es wurden 41,8 g (64% der Theorie) 4-Chlor-2-methyl-N-(chlormethansulfo)-anilin mit einer Reinheit von 97% erhalten.

## Beispiel 2 (Vergleichsbeispiel)

In einem 2-l-Vierhalskolben wurden 200 ml Wasser, 63,8 g 3-Chloranilin und 51 g Salzsäure (36%ig) vorgelegt und 10 Minuten gerührt. Das Gemisch wurde auf 5°C abgekühlt und mit 15%iger Sodalösung auf einen pH-Wert von 2 eingestellt.

Bei dieser Temperatur wurde innerhalb von 2 Stunden eine 68%ige Lösung (142 g) von Chlormethansulfochlorid in Dichlorpropan zugetropft. Der pH-Wert wurde durch gleichzeitige Zudosierung von 15%iger Sodalösung bei einem Wert von 2 gehalten. Anschließend wurde bei 25°C noch 15 Stunden nachgerührt, wobei der pH-Wert durch weitere Zugabe von Sodalösung konstant bei einem Wert von 2 gehalten wurde.

Anschließend wurde das Reaktionsgemisch auf 45°C erwärmt und mit 200 ml 1,2-Dichlorpropan versetzt. Die wäßrige Phase wurde abgetrennt und verworfen, die organische Phase 5mal mit 100 ml 15%iger Salzsäure und danach 3mal mit 100 ml Wasser gewaschen. Anschließend wurde die organische Phase am Rotationsverdampfer einer Vakuumdestillation (ca. 50 mbar) bei 60°C unterworfen. Es wurde ein Rückstand erhalten, der aus 3-Chlor-N(chlormethansulfo)-anilin bestand. Erhalten wurden 102 g (85,4% der Theorie) eines hellgelben Produkts mit einer Reinheit von 99,9%.

## Beispiel 3

In einem 2-l-Vierhalskolben wurden 200 ml Wasser, 2,25 g Benzyldodecylmethylammoniumchlorid, 63,8 g 3-Chloranilin und 51 g Salzsäure (36%ig) vorgelegt und 10 Minuten gerührt. Der Kolbeninhalt wurde auf 5°C abgekühlt und mit 15%iger Sodalösung auf einen pH-Wert von 2 eingestellt.

Bei dieser Temperatur wurde innerhalb von 2 Stunden eine 68%ige Lösung (142 g) von Chlormethansulfochlorid in 1,2-Dichlorpropan zugetropft.

Die Reaktion wurde analog Beispiel 2 durchgeführt.

Es wurden 118,5 g (99,2% der Theorie) 3-Chlor-N(chlormethansulfo)-anilin mit einer Reinheit von 99,9% erhalten.

Beispiel 4 (Vergleichsbeispiel)

In einem 2-l-Vierhalskolben wurden 400 g Wasser, 61 g Salzsäure (36%ig) und 110 g o-Aminodiphenylether vorgelegt und 20 Minuten bei 45°C gerührt. Das Gemisch wurde auf 3°C abgekühlt und der pH-Wert durch Zugabe einer 17%igen Sodalösung auf 1,4 eingestellt.

Bei dieser Temperatur wurde innerhalb von 4 Stunden eine Lösung von 119 g Chlormethansulfochlorid in 50 g Dichlorethan zugetropft. Der pH-Wert wurde durch gleichzeitige Zudosierung einer 17%igen Sodalösung konstant bei einem Wert von 1,4 gehalten. Anschließend wurde bei 20°C noch 25 Stunden nachgerührt, wobei der pH-Wert durch weitere Zugabe von Sodalösung konstant gehalten wurde. Der Verbrauch an 17%iger Sodalösung betrug 408 g.

Anschließend wurde der Kolbeninhalt auf 45 bis 50°C erwärmt und mit 150 ml Dichlorethan versetzt. Die wäßrige Phase wurde abgetrennt und verworfen, die organische Phase 5mal mit je 100 ml 15%iger Salzsäure und danach 2mal mit je 100 ml Wasser extrahiert. Dabei entfernte man nicht umgesetzten o-Aminodiphenylether. Anschließend wurde aus der organischen Phase durch Vakuumdestillation (ca. 110 mbar) bei 50°C das Lösungsmittel entfernt. Der Destillationsrückstand bestand aus N-(Chlormethansulfo)-2-aminodiphenylether. Er hatte eine hellgelbe Farbe.

Die Ausbeute betrug 146 g (82,5% der Theorie); Reinheit 99,8%.

Beispiel 5

In einem 2-l-Vierhalskolben wurden 400 g Wasser, 61 g Salzsäure (36%ig), 110 g o-Aminodiphenylether vorgelegt und das Gemisch 20 Minuten bei 45°C gerührt. Anschließend wurde das Gemisch auf 3°C abgekühlt und mit 17%iger Sodalösung versetzt, so daß sich ein pH-Wert von 1,4 einstellte. Danach setzte man dem Gemisch 5,5 g einer 50%igen wäßrigen Lösung von Benzyldodecyldimethylammoniumchlorid zu und rührte 10 Minuten.

Bei einer Temperatur von ca. 5°C wurde innerhalb von 4 Stunden eine Lösung von 119 g Chlormethansulfochlorid in 50 g Dichlorethan zugetropft. Der pH-Wert wurde durch gleichzeitige Zudosierung einer 17%igen Sodalösung bei einem pH-Wert von 1,4 gehalten. Anschließend wurde bei 20°C noch 25 Stunden nachgerührt, wobei der pH-Wert von 1,4 durch weitere Zugabe von Sodalösung konstant gehalten wurde.

Die Aufarbeitung des Reaktionsgemisches erfolgte analog Beispiel 4. Das Reaktionsprodukt hatte eine hellgelbe Farbe.

Es wurden 173,3 g (97,8% der Theorie) an N-(Chlormethansulfo)-2-aminodiphenylether mit einer Reinheit von 99,9% erhalten.

Beispiel 6

In einem 8 $m^3$ stahlemaillierten Rührkessel wurden 100 kg Wasser und 65 kg Triisopropanolamin vorgelegt. Der Kessel wurde evakuiert und durch Einleiten von Ammoniak wurde ein Überdruck von 0,2 bar eingestellt. Bei 22 bis 24°C wurde unter Kühlung ein Gemisch von 1113 kg reines n-Alkan-$C_{15}$-sulfochlorids und 1537 kg n-Paraffin ($C_{15}$) innerhalb von 8 Stunden zudosiert. Gleichzeitig wurde so viel Ammoniak eingeleitet, daß stets ein pH-Wert von 9,5 bis 10,5 vorhanden war. Der Druck wurde bei 0,1 bis 0,3 bar gehalten. Mit diesen pH- und Druckwerten wurde die Zulaufgeschwindigkeit des $C_{15}$-n-Alkansulfochlorids gekoppelt. Anschließend wurde der Ansatz 3 bis 5mal mit je 1200 l Wasser gewaschen und dann einer Vakuumdestillation unterworfen. Der Destillationsrückstand bestand aus 1034 kg (99% der Theorie) n-Pentadecylsulfonamid mit einer Reinheit von 99,5%.

Die Reaktionszeit betrug 8 Stunden.

Beispiel 7 (Vergleichsbeispiel)

In einem 8 $m^3$ stahlemaillierten Rührwerkskessel wurden 100 kg Wasser vorgelegt. Der Kessel wurde evakuiert, und durch Einleiten von Ammoniak stellte man einen Druck von 0,2 bar ein. Bei 22 bis 24°C wurde unter Kühlung innerhalb von 60 Stunden ein Gemisch von 1113 kg eines $C_{15}$-n-Alkansulfochlorids und 1537 kg $C_{15}$-n-Alkan zudosiert. Gleichzeitig wurde so viel Ammoniak eingeleitet, daß der pH-Wert stets bei 9,5 bis 10,5 lag und sich ein Druck von 0,1 bis 0,3 bar einstellte. Mit diesen pH- und

Druckwerten wurde die Zulaufgeschwindigkeit von $C_{15}$-Alkansulfochlorid gekoppelt.

Die Aufarbeitung erfolgte wie in Beispiel 6 beschrieben.

Man erhielt 1024 kg (98% der Theorie) an n-Pentadecylsulfonamid mit einer Reinheit von 99,4%.

Die Reaktionszeit betrug 60 Stunden.

## Beispiel 8

In einem 8 $m^3$ stahlemaillierten Rührwerkskessel wurden 100 kg Wasser und 60 kg Triisopropanolamin vorgelegt. Der Kessel wurde evakuiert, und durch Einleiten von Ammoniak wurde ein Druck von 0,2 bar eingestellt. Bei einer Temperatur von 22 bis 25°C wurde unter Kühlung innerhalb von 8 Stunden ein Gemisch aus 908 kg $C_{11}$-n-Alkan-sulfochlorid und 1254 kg $C_{11}$-n-Paraffin zudosiert. Gleichzeitig wurde so viel Ammoniak eingeleitet, daß sich ein pH-Wert von 9,5 bis 10,5 und ein Druck von 0,1 bis 0,3 bar einstellten. Mit diesen pH- und Druckwerten wurde die Zulaufgeschwindigkeit von $C_{11}$-n-Alkansulfochlorid gekoppelt.

Die Aufarbeitung erfolgte analog Beispiel 6.

Man erhielt 823 kg (98% der Theorie) an n-Undecylsulfonamid mit einer Reinheit von 99,6%.

Die Reaktionszeit betrug 8 Stunden.

## Vergleichsbeispiel

Es wurde analog Beispiel 8 gearbeitet, nur wurde dem Reaktionsgemisch kein Triisopropanolamin zugesetzt.

Man erhielt 819 kg (97,9% der Theorie) an n-Undecylsulfonamid mit einer Reinheit von 99,3%.

Die Reaktionszeit betrug allerdings 52 Stunden.

## Beispiel 9

In einem 2-l-Vierhalskolben wurden 200 g Wasser, 54 g Salzsäure (36%ig) und 46,5 g Anilin vorgelegt. Die Umsetzung wurde wie in Beispiel 1 beschrieben, durchgeführt. Als Reaktionspartner von Anilin wurde Benzolsulfonsäurechlorid (164 g einer 70%igen Lösung in Dichlorpropan) verwendet.

Als Katalysator wurden 4,5 g einer 50%igen wäßrigen Lösung von Benzyldodecyldimethylammoniumchlorid eingesetzt. Nach Ende der Umsetzung wurde der Überschuß von Benzolsulfonsäurechlorid bei 60°C mit Natronlauge verseift. Anschließend wurde die organische Phase 5mal mit je 80 ml 15%iger Salzsäure und dann noch 2mal mit je 80 ml Wasser extrahiert.

Durch Vakuumdestillation bei 60°C entfernte man das Lösungsmittel.

Es wurden 114,9 g (99% der Theorie) an N-(benzolsulfo)-anilin mit einer Reinheit von 99,8% erhalten.

## Vergleichsbeispiel

Es wurde analog Beispiel 9 gearbeitet, nur wurde kein Benzyldodecyldimethylammoniumchlorid bei der Umsetzung verwendet.

Es wurden 106 g (91,1% der Theorie) an N-(benzolsulfo)-anilin mit einer Reinheit von 99,7% erhalten.

**Patentansprüche**

1. Verfahren zur Herstellung von Sulfonamiden durch Umsetzung von Sulfonsäurehalogeniden mit Ammoniak oder primären oder sekundären Aminen, dadurch gekennzeichnet, daß man die Umsetzung in einem Zweiphasensystem, bestehend aus einer wäßrigen Phase und einer organischen Phase, in Gegenwart von 0,1 bis 12 Gew.-% eines tertiären Amins und/oder quarternären Ammoniumsalzes, bezogen auf das eingesetzte Sulfonsäurehalogenid, durchführt.

2. Verfahren nach Anspruch 1, dadurch gekennzeichnet, daß man als tertiäre Amine solche der allgemeinen Formel

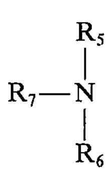

in der
$R_5$, $R_6$, $R_7$ unabhängig voneinander für einen gegebenenfalls substituierten Alkyl-, Cycloalkyl-, Aralkyl- oder Arylrest stehen oder jeweils zwei der Reste $R_5$, $R_6$, $R_7$ zusammen mit dem Stickstoffatom einen Heterocyclus bilden,
einsetzt.

3. Verfahren nach Ansprüchen 1 und 2, dadurch gekennzeichnet, daß man Tris-(2-hydroxypropyl)-amin einsetzt.

4. Verfahren nach Anspruch 1, dadurch gekennzeichnet, daß man als quartäre Ammoniumsalze solche der allgemeinen Formel

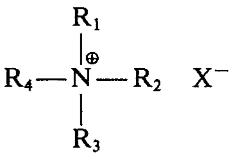

in der
$R_1$, $R_2$, $R_3$ und $R_4$ unabhängig voneinander einen gegebenenfalls substituierten Alkyl-, Aralkyl- oder Arylrest bedeuten oder wobei jeweils zwei der Reste $R_1$, $R_2$, $R_3$ und $R_4$ gemeinsam mit dem Stickstoff einen Heterocyclus bilden und
$X^-$ für ein Halogen- oder Hydroxyl-Ion steht,
einsetzt.

5. Verfahren nach Anspruch 1 und 4, dadurch gekennzeichnet, daß man Benzyldodecyldimethylammoniumchlorid einsetzt.

6. Verfahren nach Anspruch 1 bis 5, dadurch gekennzeichnet, daß man die tertiären Amine und/oder quarternären Ammoniumsalze in Mengen von 0,5 bis 5,5 Gew.-%, bezogen auf das Gewicht der eingesetzten Sulfonsäurehalogenide, einsetzt.

7. Verfahren nach Ansprüchen 1 bis 6, dadurch gekennzeichnet, daß man die Umsetzung bei Temperaturen von −15 bis 180° C durchführt.

**Claims**

1. Process for the preparation of sulphonamides by reaction of sulphonic acid halides with ammonia or primary or secondary amines, characterised in that the reaction is carried out in a two-phase system consisting of an aqueous phase and an organic phase, in the presence of 0.1 to 12% by weight of a tertiary amine and/or quaternary ammonium salt, relative to the sulphonic acid halide employed.

2. Process according to Claim 1, characterised in that the tertiary amines employed are those of the general formula

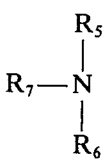

in which
$R_5$, $R_6$ and $R_7$ independently of one another represent an optionally substituted alkyl, cycloalkyl, aralkyl or aryl radical or in each case two of the radicals $R_5$, $R_6$ and $R_7$, together with the nitrogen atom, form a heterocyclic ring.

3. Process according to Claims 1 and 2, characterised in that tris-(2-hydroxypropyl)-amine is employed.

4. Process according to Claim 1, characterised in that the quaternary ammonium salts employed are those of the general formula

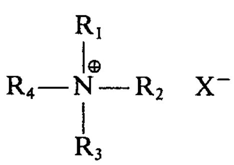

in which
$R_1$, $R_2$, $R_3$ and $R_4$ independently of one another denote an optionally substituted alkyl, aralkyl or aryl radical, or wherein in each case two of the radicals $R_1$, $R_2$, $R_3$ and $R_4$ together with the nitrogen form a heterocyclic ring, and

X⁻ represents a halogen ion or hydroxyl ion.

5. Process according to Claims 1 and 4, characterised in that benzyldodecyldimethylammonium chloride is employed.

6. Process according to Claims 1 to 5, characterised in that the teriary amines and/or quaternary ammonium salts are employed in amounts of 0.5 to 5.5% by weight, relative to the weight of sulphonic acid halides employed.

7. Process according to Claims 1 to 6, characterised in that the reaktion is carried out at temperatures of −15 to 180° C.

**Revendications**

1. Procédé de préparation de sulfonamides par réaction d'halogénures d'acides sulfoniques avec de l'ammoniac ou des amines primaires ou secondaires, caractérisé en ce qu'on effectue la réaction dans un système à deux phases constitué d'une phase aqueuse et d'une phase organique, en présence de 0,1 à 12% en poids d'une amine tertiaire et/ou d'un sel d'ammonium quaternaire, calculé sur l'halogénure d'acide sulfonique utilisé.

2. Procédé suivant la revendication 1, caractérisé en ce que, comme amines tertiaires, on utilise celles répondant à la formule générale:

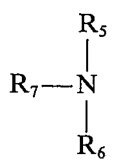

dans laquelle
$R_5$, $R_6$ et $R_7$ représentent chacun indépendamment l'un de l'autre un groupe alkyle, un groupe cycloalkyle, un groupe aralkyle ou un groupe aryle éventuellement substitué ou chaque fois deux des radicaux $R_5$, $R_6$ et $R_7$, ensemble avec l'atome d'azote, forment un noyau hétérocyclique.

3. Procédé suivant les revendications 1 et 2, caractérisé en ce qu'on utilise la tris-(2-hydroxypropyl)-amine.

4. Procédé suivant la revendication 1, caractérisé en ce que, comme sels d'ammonium quaternaire, on utilise ceux répondant à la formule générale:

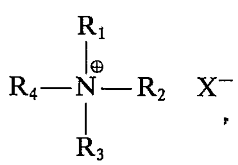

dans laquelle
$R_1$, $R_2$, $R_3$ et $R_4$ représentent chacun indépendamment l'un de l'autre un groupe alkyle, un groupe aralkyle ou un groupe aryle éventuellement substitué ou chaque fois deux des radicaux $R_1$, $R_2$, $R_3$ et $R_4$, ensemble avec l'atome d'azote, forment un noyau hétérocyclique, et
X⁻ représente un ion halogène ou un ion hydroxy.

5. Procédé suivant les revendications 1 et 4, caractérisé en ce qu'on utilise le chlorure de benzyl-dodécyl-diméthylammonium.

6. Procédé suivant les revendications 1 à 5, caractérisé en ce qu'on utilise les amines tertiaires et/ou les sels d'ammonium quaternaire en quantités de 0,5 à 5,5% en poids, calculé sur le poids des halogénures d'acides sulfoniques utilisés.

7. Procédé suivant les revendications 1 à 6, caractérisé en ce qu'on effectue la réaction à des températures de −15 à 180° C.